# EUROPEAN PATENT APPLICATION

(11) **EP 4 566 664 A1**
(43) Date of publication of application: **11.06.2025**
(21) Application number: 24810228.7
(22) Date of filing: 10.05.2024
(51) Int. Cl.: A61N 7/00

(54) **THERAPEUTIC INSTRUMENT HANDLE AND CONTROL METHOD THEREFOR, AND THERAPEUTIC INSTRUMENT**

(30) Priority: 19.05.2023 CN 202310573798; 19.05.2023 CN 202310580299
(71) Applicant: Shenzhen Peninsula Medical Group, Shenzhen, Guangdong 518000 (CN)
(72) Inventor: LI, Yanan, Shenzhen, Guangdong 518000 (CN); HU, Shuyun, Shenzhen, Guangdong 518000 (CN); LEI, Xiaobing, Shenzhen, Guangdong 518000 (CN); DING, Yi, Shenzhen, Guangdong 518000 (CN)
(74) Representative: Osterhoff, Utz
(86) International application number: PCT/CN2024/092437
(87) International publication number: WO 2024/239981

(57) **Abstract**

The present application discloses a treatment device handle, a control method thereof, and a treatment device. The treatment device includes an interface module and a working module. The control method for the treatment device handle includes: obtaining the kind of the treatment tip currently connected to the interface module; and controlling the working module to output the driving signal to the treatment tip through the interface module according to the kind of the treatment tip to drive the treatment tip to work.

## Description

The present application claims priority to Chinese Patent Application No. 202310573798.8 and Chinese Patent Application No. 202310580299.1, both filed on May 19, 2023, the entire contents of which are incorporated herein by reference.

### TECHNICAL FIELD

The present application relates to the technical field of treatment devices, and in particular to a treatment device handle and a control method thereof, and a treatment device.

### BACKGROUND

Many existing treatment devices can only use a single treatment method when treating the human body. If users want to experience different treatment methods, they need to purchase different kinds of treatment devices, which increases the user's purchase cost and reduces the user experience.

### SUMMARY

The present application aims to provide a treatment device handle and a control method thereof, and a treatment device, to solve the problem of reducing user experience caused by the single treatment method of the treatment device.

In order to achieve the above purpose, the present application provides a treatment device handle, which includes:
an interface module, being connected with at least one kind of treatment tip;
a working module electrically coupled to the interface module; and
a main control module coupled to the working module, the main control module is configured to control the working module to drive the at least one kind of treatment tip through the interface module.

In an embodiment of the present application, the interface module includes at least one communication interface and at least one energy interface;
the energy interface is coupled to the working module and is configured to access a signal terminal of each treatment tip; and
the communication interface is coupled to the main control module and is configured to access a communication terminal of the treatment tip; and in response to that the interface module is connected with each treatment tip, the communication terminal of each treatment tip is configured to establish an electrical connection path with the main control module through the communication interface.

In an embodiment of the present application, a plurality of the energy interfaces are provided, and each energy interface is connected with one kind of treatment tip; and
the working module includes a plurality of sub-working modules, and an output end of each sub-working module is coupled to one of the plurality of energy interfaces.

In an embodiment of the present application, at least one energy interface is provided, and each energy interface is configured to access at least two kinds of treatment tips;
the working module includes a switch module and a plurality of sub-working modules; the switch module is coupled to output ends of the plurality of sub-working modules and at least one energy interface, and a controlled terminal of the switch module is coupled to the main control module; or
the working module includes a plurality of sub-working modules, and the plurality of sub-working modules are coupled to the main control module.

In an embodiment of the present application, the working module includes a drive module and a switch module; the switch module is coupled to an output end of the drive module and at least one energy interface respectively, and a controlled end of the switch module is coupled to the main control module.

In an embodiment of the present application, the main control module is provided with a signal access terminal for accessing a trigger signal; and
the main control module is configured to control a working state of the drive module in response to receiving the trigger signal.

In an embodiment of the present application, the main control module is provided with a signal access terminal for accessing a trigger signal; and
the main control module is configured to control a working state of a sub-working module corresponding to the treatment tip in response to receiving the trigger signal.

In an embodiment of the present application, the interface module includes a plurality of energy interfaces, and each of the energy interfaces is configured to access one kind of treatment tip;
the working module includes a plurality of sub-working modules, and an output end of each sub-working module is coupled to one of the plurality of energy interfaces; and
the main control module is provided with a signal access terminal for accessing a trigger signal, and is configured to control a working state of a sub-working module according to the trigger signal in response to receiving the trigger signal.

In an embodiment of the present application, the interface module includes at least one energy interface, and each energy interface is configured to access at least two kinds of treatment tips;
the working module includes a switch module and a plurality of sub-working modules; the switch module is coupled to output ends of the plurality of sub-working modules and the at least one energy interface, and a controlled end of the switch module is coupled to the main control module; and
the main control module is provided with a signal access terminal for accessing a trigger signal, and is configured to control operation of the switch module and control a working state of a sub-working module according to the trigger signal in response to receiving the trigger signal.

In an embodiment of the present application, the interface module includes at least one energy interface; the working module includes a drive module and a switch module; the switch module is coupled to an output end of the drive module and at least one energy interface respectively, and a controlled end of the switch module is coupled to the main control module; and
the main control module is provided with a signal access terminal for accessing a trigger signal, and is configured to control operation of the switch module and a working state of the drive module in response to receiving the trigger signal.

In an embodiment of the present application, the plurality of sub-working modules includes at least two of an ultrasonic energy output module, a radio frequency energy output module, a low frequency energy output module, a medium frequency energy output module and a light energy output module.

In an embodiment of the present application, the treatment device handle further includes:
a trigger module is coupled to a signal access terminal of the main control module, in response to that the interface module is connected to the treatment tip, the trigger module is triggered to output a access signal to the main control module; or
the trigger module is configured to output a trigger signal to the main control module in response to that the trigger module is triggered by a user.

The present application also provides a control method for a treatment device handle, the treatment device handle includes an interface module and a working module, and the method includes:
obtaining kind of each of a plurality of treatment tips currently connected to the interface module; and
according to the kind of each treatment tip, controlling the working module to output a driving signal to the treatment tip through the interface module to drive the treatment tip to work.

In an embodiment of the present application, the treatment device handle further includes a trigger module, and obtaining the kind of each treatment tip currently connected to the interface module includes:
obtaining a trigger signal sent from the trigger module;
determining the kind of each treatment tip currently connected to the interface module according to the trigger signal; or
obtaining the kind of each treatment tip currently connected to the interface module includes:
   obtaining a setting signal sent from an external terminal; and
   according to the setting signal, determining the kind of the treatment tip currently connected to the interface module.

In an embodiment of the present application, obtaining the kind of the treatment tip currently connected to the interface module includes:
in response to that the treatment device handle recognizes that the interface module is connected to each treatment tip, establishing a communication connection between the treatment device handle and the treatment tip; and
obtaining treatment tip parameter information from the treatment tip, and determining the kind of the treatment tip currently connected to the interface module according to the treatment tip parameter information.

In an embodiment of the present application, the working module includes a plurality of sub-working modules; and the according to the kind of each treatment tip, controlling the working module to output the driving signal to the treatment tip through the interface module to drive the treatment tip to work includes:
according to the kind of each treatment tip, determining the sub-working module corresponding to the kind of each treatment tip; and
controlling the corresponding sub-working module to output a corresponding driving signal to the treatment tip through the interface module to drive the treatment tip to work.

In an embodiment of the present application, the interface module includes at least one energy interface for accessing at least two kinds of treatment tips, and the working module also includes a switch module; the controlling the corresponding sub-working module to output the corresponding driving signal to the treatment tip through the interface module to drive the treatment tip to work includes:
according to the kind of each treatment tip, controlling operation of the switch module to conduct a path between the sub-working module corresponding to the kind of the treatment tip and the energy interface for accessing the treatment tip; and
controlling the corresponding sub-working module to output the corresponding driving signal to the treatment tip through the switch module and the energy interface to drive the treatment tip to work.

In an embodiment of the present application, the interface module includes at least one energy interface for accessing the treatment tip, and the working module includes a switch module and a drive module; the according to the kind of each treatment tip, controlling the working module to output the driving signal to the treatment tip through the interface module to drive the treatment tip to work includes:
according to the kind of each treatment tip, controlling operation of the switch module to conduct a path between the drive module and the energy interface for accessing the treatment tip; and
controlling the drive module to output the driving signal corresponding to the kind of each treatment tip to the treatment tip through the switch module and the energy interface to drive the treatment tip to work.

In an embodiment of the present application, before the obtaining the kind of each treatment tip currently connected to the interface module, the method further includes:
obtaining access status information of the treatment tip currently connected to the interface module;
according to the access status information, in response to determining that the treatment tip is completely connected to the interface module, obtaining the kind of each treatment tip currently connected to the interface module; or
according to the access status information, in response to determining that the treatment tip is not completely connected to the interface module, prompting or controlling the working module to stop working.

The present application also provides a treatment device, including a plurality of treatment tips and a treatment device handle as described above, the plurality of treatment tips are connected to the treatment device handle according to the control method for the treatment device handle as described above, and an output driving signal is obtained and controlled; the plurality of treatment tips includes at least two of an ultrasonic treatment tip, a low-frequency treatment tip, a medium-frequency treatment tip and a light treatment tip.

The control method for the treatment device handle of the present application includes: obtaining a kind of a treatment tip currently connected to the interface module; and according to the kind of the treatment tip, controlling the working module to output a driving signal to the treatment tip through the interface module to drive the treatment tip to work. In this way, in practical applications, users can access and drive different kinds of treatment tips through the treatment device handle to experience different kinds of treatment methods without purchasing an additional set of treatment devices. The present application realizes the compatibility of the treatment device handle with different kinds of treatment tips, thereby effectively improving the user's experience and convenience in using the treatment device.

### BRIEF DESCRIPTION OF THE DRAWINGS

In order to more clearly explain technical solutions in the embodiments of the present application or in the related art, accompanying drawings required in the description of the embodiments or the related art will be briefly introduced below. Obviously, the accompanying drawings in the following description are only some embodiments of the present application. For those skilled in the art, other drawings can be obtained based on the structures shown in these drawings without creative efforts.
FIG. 1 is a schematic flowchart of a control method for a treatment device handle according to an embodiment of the present application.
FIG. 2 is a schematic flowchart of the control method for the treatment device handle according to another embodiment of the present application.
FIG. 3 is a schematic flowchart of the control method for the treatment device handle according to another embodiment of the present application.
FIG. 4 is a schematic flowchart of the control method for the treatment device handle according to another embodiment of the present application.
FIG. 5 is a schematic flowchart of the control method for the treatment device handle according to another embodiment of the present application.
FIG. 6 is a schematic flowchart of the control method for the treatment device handle according to another embodiment of the present application.
FIG. 7 is a schematic diagram of functional modules of the treatment device handle according to an embodiment of the present application.
FIG. 8 is a schematic diagram of functional modules of the treatment device handle according to an embodiment of the present application.
FIG. 9 is a schematic diagram of functional modules of the treatment device handle according to an embodiment of the present application.
FIG. 10 is a schematic diagram of functional modules of the treatment device handle according to an embodiment of the present application.
FIG. 11 is a schematic diagram of functional modules of the treatment device handle according to an embodiment of the present application.
FIG. 12 is a schematic diagram of functional modules of the treatment device handle according to an embodiment of the present application.

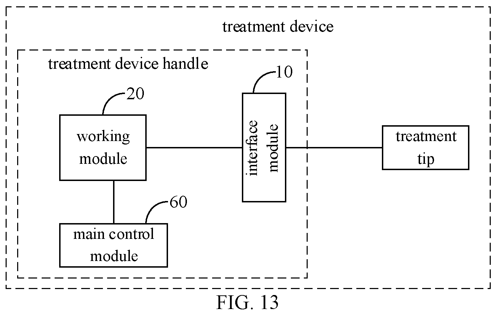

FIG. 13 is a schematic diagram of functional modules of the treatment device handle according to an embodiment of the present application.
FIG. 14 is a schematic diagram of functional modules of the treatment device handle according to an embodiment of the present application.
FIG. 15 is a schematic diagram of functional modules of the treatment device handle according to an embodiment of the present application.
FIG. 16 is a schematic diagram of functional modules of the treatment device handle according to an embodiment of the present application.
FIG. 17 is a schematic diagram of functional modules of the treatment device handle according to an embodiment of the present application.
FIG. 18 is a schematic diagram of functional modules of the treatment device handle according to an embodiment of the present application.
FIG. 19 is a schematic diagram of functional modules of the treatment device handle according to an embodiment of the present application.
FIG. 20 is a schematic diagram of functional modules of the treatment device handle according to an embodiment of the present application.
FIG. 21 is a schematic diagram of functional modules of the treatment device handle according to an embodiment of the present application.
FIG. 22 is a schematic structural diagram of a treatment tip of the treatment device according to an embodiment of the present application.
FIG. 23 is a schematic structural diagram of a treatment device handle of the treatment device according to an embodiment of the present application.
FIG. 24 is a schematic structural diagram of the assembly of the treatment tip and the treatment device handle of the treatment device according to an embodiment of the present application.

### Description of reference signs:

10. interface module; 20. working module; 30. control device; 40. trigger module; 50. communication module; 60. main control module; 11. energy interface; 12. communication interface; 21. sub-working module; 22. switch module; 23. drive module; 100. treatment tip; 200. treatment device handle.

### DETAILED DESCRIPTION OF THE EMBODIMENTS

The technical solutions in the embodiments of the present application will be clearly and completely described below with reference to the accompanying drawings in the embodiments of the present application. Obviously, the described embodiments are only some rather than all of the embodiments of the present application. Based on the embodiments in the present application, all other embodiments obtained by those skilled in the art without creative efforts fall within the scope of the present application.

It should be noted that all directional indications (such as up, down, left, right, front, back...) in the embodiments of the present application are only used to explain the relative positional relationship between components in a specific posture (as shown in the drawings), movement conditions, etc. If the specific posture changes, the directional indication will also change accordingly.

In the present application, unless otherwise clearly stated and limited, terms "connection", "fix", etc. should be understood in a broad sense. For example, "fix" can be a fixed connection, a detachable connection, or an integral body; it can be a mechanical connection or an electrical connection; it can be a direct connection or an indirect connection through an intermediate medium; it can be an internal connection between two elements or an interactive relationship between two elements, unless otherwise clearly limited. For those skilled in the art, the specific meanings of the above terms in the present application can be understood according to specific circumstances.

In addition, descriptions involving "first", "second", etc. in the present application are for descriptive purposes only and cannot be understood as indicating or implying their relative importance or implicitly indicating the number of indicated technical features. Therefore, features defined as "first" and "second" can explicitly or implicitly include at least one of these features. In addition, the meaning of "and/or" appearing in the entire text is to include three parallel solutions, taking A and/or B as an example, including solution A, or solution B, or a solution that both A and B satisfy at the same time. In addition, the technical solutions in the various embodiments can be combined with each other, but it must be based on what those skilled in the art can implement. When the combination of technical solutions is contradictory or cannot be realized, it should be considered that such a combination of technical solutions does not exist, nor is it within the scope of the present application.

Many existing treatment devices can only use a single treatment method when treating the human body. If users want to experience different treatment methods, they need to purchase different kinds of treatment devices, which increases the user's purchase cost and reduces the user experience.

To this end, the present application proposes a treatment device handle, which includes an interface module 10, a working module 20 and a main control module 60.

The interface module 10 is configured to connect at least one kind of treatment tip.

The working module 20 is coupled to the interface module 10.

The main control module 60 is coupled to the working module 20 and is configured to control the working module 20 to drive the connected treatment tip through the interface module 10.

It should be understood that there are often many kinds of treatment tips in the treatment device, such as ultrasonic treatment tips, radio frequency treatment tips, and light treatment tips.

In this embodiment, an installation structure is arranged on the treatment device handle, for installing a fixedly connected treatment tip, and the interface module 10 is arranged in the installation structure, so that when the treatment tip is connected to the installation structure, multiple connection terminals on the treatment tip is connected to the interface module 10 to realize electrical connection with each other.

In this embodiment, the interface module 10 is implemented using standard connectors, such as female connectors, male connectors, and inter-board connectors, or using multiple conductive components, such as metal pins and metal electrode posts, metal electrode sheets.

In an embodiment, the interface module 10 is provided with a plurality of the above-mentioned connectors or conductive parts and other connecting devices for realizing electrical connection functions. Each connecting device is connected to one kind of treatment tip.

In an embodiment, the number of devices that implement electrical connection functions such as connectors or conductive parts in the interface module 10 is at least one and no more than the number of kinds of treatment tips. In other words, the structures of the connections for connecting the interface module 10 on some different kinds of treatment tips are exactly the same, that is, the treatment tips of these two kinds share a certain connection device on the interface module 10, thereby effectively simplifying the structure of the treatment device handle, and reducing the cost and process difficulty of manufacturing the treatment device handle.

In this embodiment, the main control module 60 is implemented by a main controller, such as micro controller unit (MCU), digital signal processing (DSP), field programmable gate array (FPGA), programmable logic controller (PLC), system on chip (SOC), etc.

In this embodiment, the main control module 60 controls the working module 20 to output corresponding driving signals to drive the connected treatment tip. In an embodiment, the working module 20 includes a variety of corresponding sub-working modules 21 according to the actual kind of the treatment tip, such as at least two of an ultrasonic energy output module, a radio frequency energy output module and a light energy output module. When the treatment tip is connected to the interface module 10, the main control module 60 controls the operation of the sub-working module 21 corresponding to the kind of the connected treatment tip, so as to output the corresponding driving signal to the treatment tip to make the connected treatment tip work. For example, for an ultrasonic probe, the main control module 60 controls the ultrasonic energy output module to output an ultrasonic driving signal to the ultrasonic probe through the interface module 10, so that after receiving the ultrasonic driving signal, the ultrasonic probe emits corresponding ultrasonic waves to act on the human body.

In an embodiment, the working module 20 can also be directly implemented using an adjustable power supply module, such as a programmable power supply chip, a direct current to direct current (DC-DC) circuit composed of multiple switch tubes, etc. When the treatment tip is connected to the interface module 10, the main control module 60 controls the adjustable power supply module to output an operating voltage (i.e., a driving signal) corresponding to the kind of the treatment tip to the treatment tip, so that the treatment tip is powered on. For example, the working voltage required by the currently connected light treatment tip is 12V. After the light treatment tip is connected, the main control module 60 controls the adjustable power supply module to output a working voltage of 12V to the light treatment tip through the interface module 10, to power the light treatment tip. At this time, the user can operate the trigger on the light treatment tip to cause the light treatment tip to emit corresponding light signals to act on the human body.

It can be understood that in the present application, the main control module 60 communicates with the connected treatment tip via the interface module 10 to determine the kind of the treatment tip and perform subsequent actions, or determines the kind of the currently connected treatment tip according to external signals and perform subsequent actions.

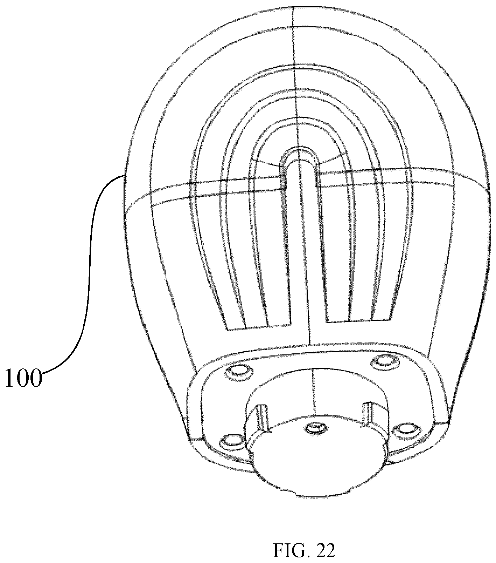

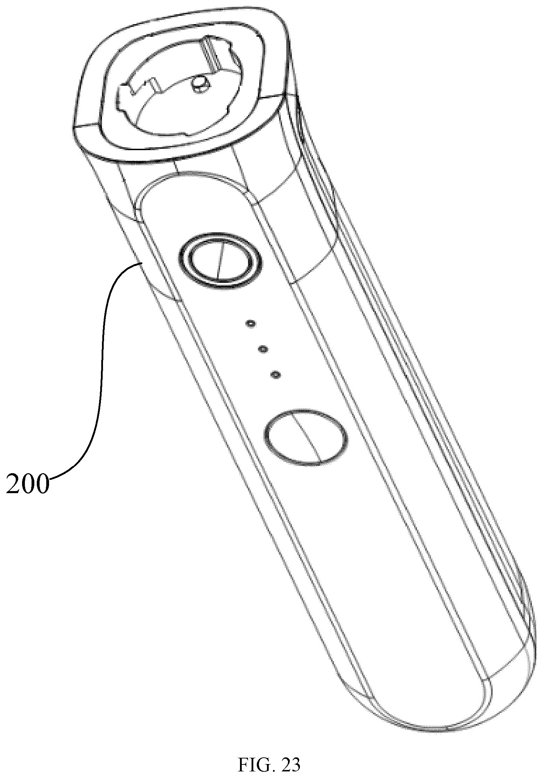

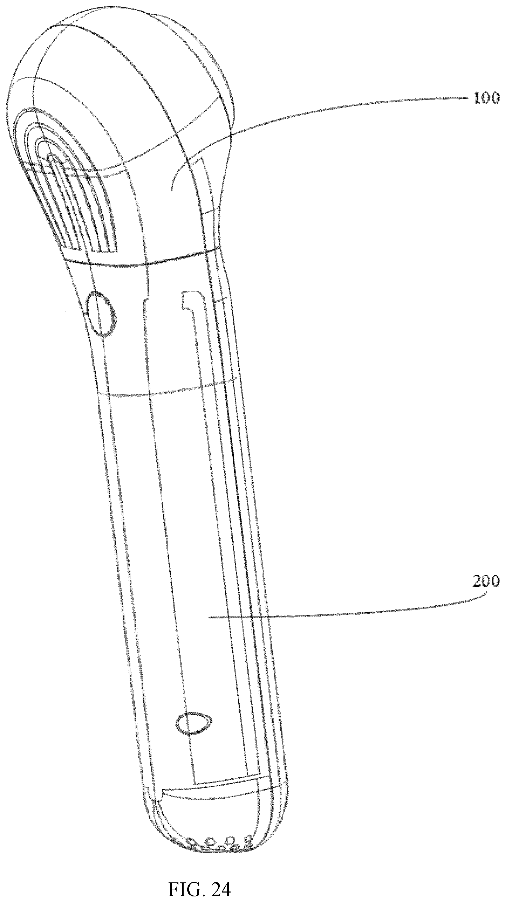

Through the above settings, as shown in FIG. 22 to FIG. 24, the treatment device handle 200 of the present application is compatible with different kinds of treatment tips 100, and after the different kinds of treatment tips 100 are connected, the working module 20 is controlled to drive different kinds of treatment tips 100, so as to make the treatment tip 100 work, thereby bringing different treatment mode experiences to the user. In this way, in practical applications, users only need to purchase the treatment device handle 200 in the present application and multiple different kinds of treatment tips 100 matched with this handle to switch between different treatment methods without purchasing a separate treatment device for each treatment method, which greatly improves the user's experience of using the treatment tip 100.

In addition, in an embodiment of the present application, the treatment device handle also includes a trigger module 40.

The trigger module 40 is coupled to a signal access terminal of the main control module 60. When the interface module 10 is connected to the treatment tip, the trigger module 40 is triggered, so that the trigger module 40 outputs the access signal to the main control module 60.

Alternatively, when triggered by the user, the trigger signal is output to the main control module 60.

In this embodiment, the trigger module 40 is a contact trigger module, such as a micro switch, etc., and the trigger module 40 is provided in the installation structure of the treatment device handle. When the treatment tip is stably connected to the installation structure, and the signal terminal on it is stably coupled to the interface module 10, the treatment tip presses the trigger module 40 provided in the installation structure, so that the trigger module 40 outputs the access signal to the main control module 60. For example, the trigger module 40 includes a micro switch. One end of the micro switch is grounded, and the other end is coupled to a pull-up circuit and the main control module 60 respectively. When the micro switch is triggered, a low level signal is output to the main control module 60. When the micro switch is not triggered, a high level signal is output to the main control module 60. In an embodiment, the trigger module 40 can also be a non-contact trigger module, such as a Hall sensor module. A magnet is provided at the bottom of the treatment tip. When the treatment tip is connected to the installation structure, the Hall sensor module detects the magnetic field formed by the gradually approaching magnet on the treatment tip, and output the access signal with a corresponding voltage value. The main control module 60 confirms that the treatment tip has been fully connected to the handle of the treatment tip when the voltage value of the access signal reaches a preset voltage threshold. The preset voltage threshold is obtained by the research personnel through multiple tests during the development period. In this way, through the above settings, when the main control module 60 receives the output access signal, it confirms that the current treatment tip has been connected in place, and then perform subsequent actions. If the output access signal cannot be received, the main control module 60 can maintain the dormant state, thereby saving the power consumed by the treatment device handle and improving the battery life of the treatment device handle. Alternatively, the treatment device handle is also provided with a prompt module, which is a light signal prompt module, such as a light emitting diode (LED) light, a display screen, etc., or is a sound prompt module. Specifically, if it is determined that the current connection status information is that the treatment tip is not fully connected, the main control module 60 controls the prompt module to maintain the prompt state. For example, the prompt module is an LED light. When the treatment tip is not connected, the main control module 60 controls the LED light to remain on, and when it is confirmed that the treatment tip is connected, the main control module 60 controls the LED light to turn off. This serves the purpose of prompting the user whether the treatment tip is currently inserted normally.

In addition, it can be understood that if the current main control module 60 confirms that the treatment tip is not fully connected according to the connection status information, it also controls the working module to stop working. For example, the current main control module 60 outputs the low level signal to the enable end of the working module to keep the working module in a stopped working state. Thus, in practical applications, if the user mistakenly triggers the trigger switch on the treatment device handle that is coupled to the main control module 60 when the treatment tip is not connected, or mistakenly clicks the function area indicating "start working" on the external terminal, so that the external terminal emits a signal indicating "start working" to the treatment device handle, and the working module 20 in the treatment device handle will not start, that is, no electrical signal is output through the energy interface, effectively ensuring safety of the user using the treatment device.

The treatment device handle of the present application includes an interface module 10, a working module 20 and a main control module 60. The interface module 10 is configured to connect at least one kind of treatment tip, and the main control module 60 is configured to control the working module 20 to output a driving signal to the connected treatment tip through the interface module 10 to drive the connected treatment tip to work. In this way, in practical applications, users connects and drives different kinds of treatment tips through the treatment device handle of the present application to experience different kinds of treatment methods without purchasing an additional set of treatment devices. The present application realizes the compatibility of the treatment device handle with different kinds of treatment tips, thereby effectively improving the user's experience and convenience in using the treatment device.

In an embodiment of the present application, the interface module 10 includes at least one communication interface 12 and at least one energy interface 11.

The energy interface 11 is coupled to the working module 20 and is configured to connect the signal end of each treatment tip.

The communication interface 12 is coupled to the main control module 60 and is configured to connect the communication end of the treatment tip. When the interface module 10 is connected with each treatment tip, a communication terminal of each treatment tip establishes an electrical connection path with the main control module 60 through the communication interface 12.

In this embodiment, the energy interface 11 and the communication interface 12 is implemented using the standard connectors or connecting devices such as conductive parts in the above embodiments. The energy interface 11 and the communication interface 12 can also be integrated into the same standard connector, or implemented using the same conductive component. For example, through carrier communication, the same conductive component provides power supply voltage to the treatment tip while transmitting communication signals.

It can be understood that the multiple communication interfaces 12 can also be provided and correspond to multiple kinds of treatment tips one-to-one, or one or more communication interfaces 12 is compatible with the connection of the communication end of at least two kinds of treatment tips.

In this embodiment, the communication interface 12 is coupled to the main control module 60. That is, when the interface module 10 is connected with each treatment tip, the communication terminal of each treatment tip establishes an electrical connection path with the main control module 60 through the communication interface 12. In other words, a communication connection is established between the treatment tip and the main control module 60 to realize data transmission. The main control module 60 determines the kind of the current treatment tip or the driving signal required for the current treatment through communication, thereby controlling the working module 20 to perform corresponding work.

In one embodiment, multiple energy interfaces 11 are provided, and each energy interface 11 is connected with one of the plurality of the treatment tips. The working module 20 includes multiple sub-working modules 21, and an output end of each sub-working module 21 is coupled to one of the multiple energy interfaces 11.

In this embodiment, the sub-working module 21 is a drive module 23 corresponding to a certain kind of treatment tip, such as an ultrasonic energy output module, a radio frequency energy output module, a light energy output module, and so on.

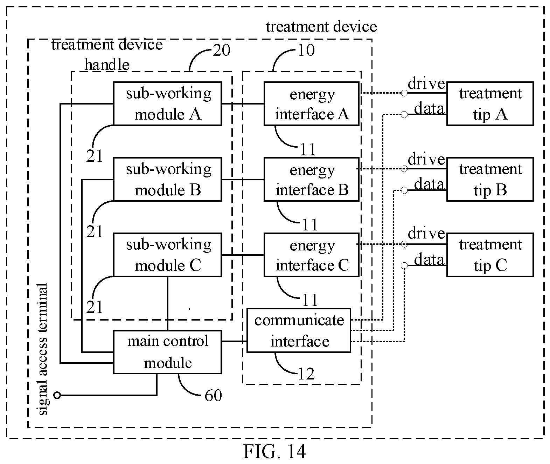

Specifically, as shown in FIG. 14, an embodiment is described as an example, in which three energy interfaces 11 are provided, and three kinds of the treatment tips are ABC. The sub-working modules 21 correspond to the three kinds ABC, and three kinds of the treatment tips correspond to the communication end of the same communication interface 12 in FIG. 14.

If the current user wants to use treatment tip A, the user installs treatment tip A in the installation structure of the treatment device handle. At this time, the energy interface 11A is coupled to the signal end of the treatment tip, and the communication interface 12 is coupled to the communication end of the treatment tip. It can be understood that the communication end of the treatment tip includes a communication power end and a communication data end. When the communication end of the treatment tip is couple to the communication interface 12, the control circuit inside can receive the working voltage from the treatment device handle via the communication power end to achieve the electricity connection between the treatment tip and the handle, and establish data communication therebetween with the main control module 60 in the treatment device handle through the communication data end. The main control module 60 outputs an inquiry signal to the outside at intervals through the communication data end. When the treatment tip is connected, the control circuit in the treatment tip receives the inquiry signal and send back a feedback signal, so that the main control module 60 determines that the current treatment tip has been connected. At this time, the main control module 60 communicates with the treatment tip to determine the kind of the treatment tip.

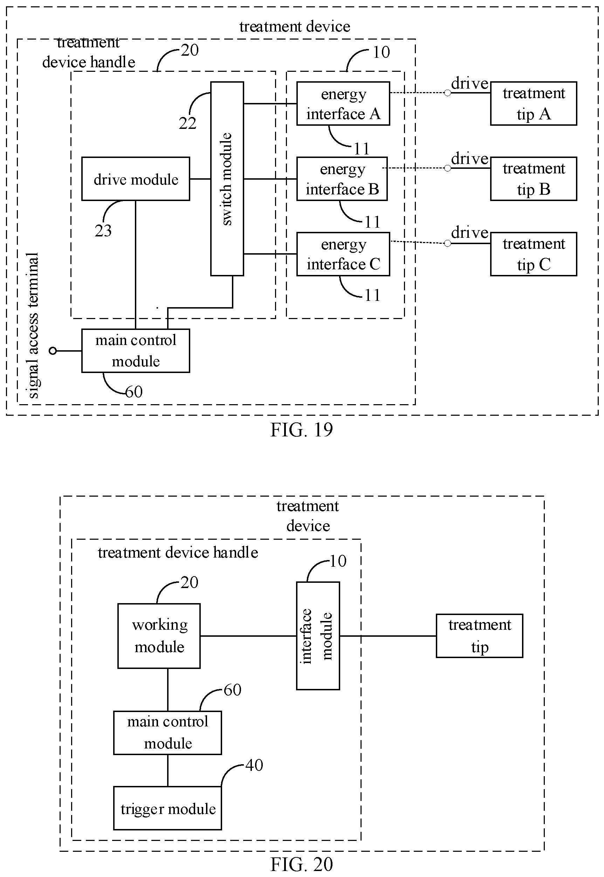

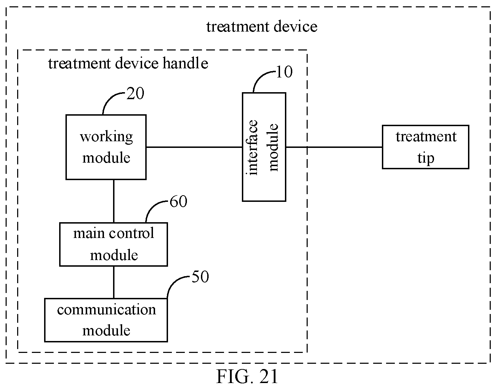

It can be understood that the main control module 60 is also provided with a signal access terminal for accessing the trigger signal, and the main control module 60 is also configured to control the sub-working module 21 corresponding to the connected treatment tip when receiving the trigger signal to be in the working state to output the driving signal to the connected treatment tip. In an embodiment, as shown in FIG. 20, the signal access terminal is coupled to the trigger module 40 on the treatment device, such as a touch screen, a button, a button array, etc., so that when the user operates the trigger module 40, the trigger module 40 outputs the trigger signal. In an embodiment, as shown in FIG. 21, the signal access terminal can also be coupled to the communication module 50, such as the WIFI communication module 50, 4G/5G communication module 50, CAN communication module 50, RS485 communication module 50, etc., thereby accessing the trigger signal transmitted from the external terminal, such as mobile phones, tablets, terminals, etc., via the communication module 50.

After receiving the trigger signal, the main control module 60 controls the corresponding sub-working module 21 to start working according to the trigger signal, so as to output the corresponding driving signal to the connected treatment tip, thereby enabling the treatment tip to start working. For example, if the current treatment tip A is a radio frequency treatment tip, the sub-working module 21 is a radio frequency drive module 23, and the user installs treatment tip A, the main control module 60 recognizes that the currently connected treatment tip is treatment tip A. Therefore, when receiving the trigger signal, the sub-working module 21 is controlled to output the corresponding radio frequency signal to the treatment tip A through the energy interface 11A. After the radio frequency signal is output to the treatment tip A, it is emitted externally through the radio frequency electrode on the treatment tip to act on the human body for treating the human body.

In addition, it can be understood that the user can also trigger the above-mentioned trigger module 40 or control an external terminal to cause the main control module 60 to stop the current sub-working module 21 from working, or to adjust the intensity of the driving signal, etc.

In another embodiment, at least one energy interface 11 is provided and the at least one energy interface 11 is configured to access at least two kinds of treatment tips.

The working module 20 includes a switch module 22 and a plurality of sub-working modules 21. The switch module 22 is coupled to the output ends of the plurality of sub-working modules 21 and at least one energy interface 11. The controlled end of the switch module 22 is coupled to the main control module 60.

The main control module 60 is configured to control the operation of the switch module 22 when the treatment tip is connected, so as to conduct the path between the sub-working module 21 corresponding to the treatment tip and the energy interface 11.

In this embodiment, the switch module 22 is composed of multiple switch tubes, such as insulated gate bipolar transistor (IGBT), metal-oxide-semiconductor (MOS), transistor, etc., or is implemented by using a gate chip.

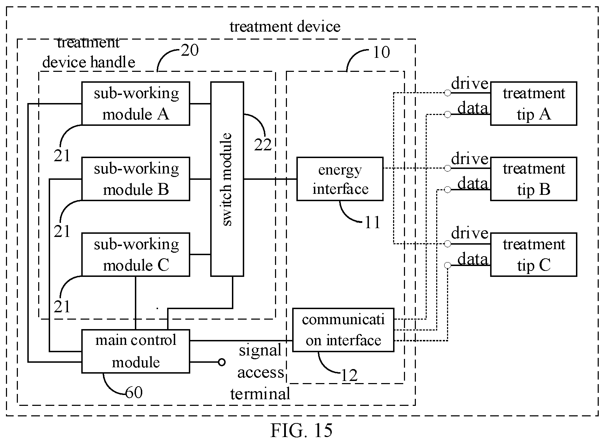

Specifically, as shown in FIG. 15, an embodiment is described as an example, where the treatment tips in FIG. 15 are of three kinds ABC, the sub-working modules 21 correspond to the three kinds ABC, and three kinds of treatment tips correspond to the signal ends of the same energy interface 11 and the three kinds of treatment tips correspond to the communication end of the same communication interface 12.

If the current user wants to use treatment tip B, the treatment tip B is connected to the installation structure of the treatment device handle. At this time, the main control module 60 confirms that the currently connected treatment tip is treatment tip B, and control the action of the switch module 22 to conduct the path between the sub-working module 21B and the energy interface 11. At this time, referring to the above embodiment, when the main control module 60 receives the trigger signal through the signal access terminal, it controls the sub-working module 20B to start working to output the driving signal to the treatment tip B through the switch module 22 and the energy interface 11, so that the treatment tip B starts to treat the user.

It can be understood that the user can also trigger the above-mentioned trigger module 40 or control an external terminal to cause the main control module 60 to stop the current sub-working module 21 from working, or to adjust the intensity of the driving signal, etc.

In another embodiment, at least one energy interface 11 is provided and is configured to connect at least two kinds of treatment tips. The work module 20 includes a plurality of sub-working modules 21, and the plurality of sub-working modules 21 are coupled to the main control module 60.

In this embodiment, at least one energy interface 11 among all energy interfaces 11 is coupled to at least two sub-working modules 21. When the main control module 60 determines the kind of the currently connected treatment tip, it controls the corresponding sub-working module 21 to output the corresponding driving signal to the treatment tip through the energy interface 11 corresponding to its electrical connection to drive the connected treatment tip to work. At the same time, other sub-working modules 21 are controlled to remain in a stopped working state.

In another embodiment, the working module 20 includes a drive module 23 and a switch module 22. The switch module 22 is coupled to the output end of the drive module 23 and at least one energy interface 11 respectively. The controlled end of the switch module 22 is coupled to the main control module 60. The main control module 60 is configured to control the operation of the switch module 22 when the treatment tip is connected, so as to conduct the path between the energy interface 11 corresponding to the treatment tip and the drive module 23.

In this embodiment, the drive module 23 is implemented using the adjustable power module in the above embodiment to provide corresponding operating voltages for different kinds of treatment tips. Alternatively, the drive module 23 can also be implemented using an integrated chip that can output different driving signals at the same time, such as a signal driving chip that can output ultrasonic signals and radio frequency signals.

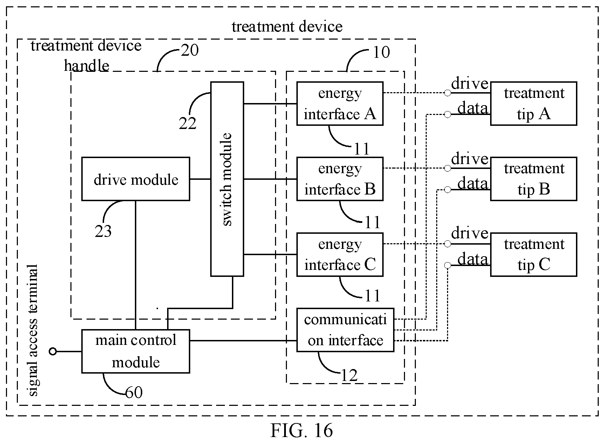

Specifically, as shown in FIG. 16, an embodiment is described as an example, where the treatment tips in FIG. 16 are of three kinds ABC, the energy interfaces 11 correspond to three kinds ABC, and three kinds of the treatment tips correspond to the communication terminals of the same communication interface 12.

When the user wants to use the treatment tip C, the treatment tip C is connected to the installation structure of the treatment device handle. At this time, communication is established between the treatment tip C and the main control module 60. The main control module 60 determines that the connected treatment tip is treatment tip C, and controls the action of the switch module 22 to conduct the path between the drive module 23 and the treatment tip C. It can be understood that, with reference to the above embodiments, the main control module 60 also has a signal access terminal for accessing the trigger signal. The main control module 60 is also configured to control the drive module 23 to be in a working state to output the corresponding driving signal to the connected treatment tip when receiving the trigger signal.

It can be understood that the user can also trigger the above-mentioned trigger module 40 or control an external terminal to cause the main control module 60 to stop the current drive module 23 from working, or to adjust the intensity of the driving signal, etc.

In addition, in practical applications, the interface module 10 only includes at least one energy interface 11 without providing a communication interface 12. In an embodiment of the present application, the interface module 10 includes multiple energy interfaces 11, each of energy interfaces 11 is configured to access one kind of treatment tip.

The working module 20 includes a plurality of sub-working modules 21, and the output end of each sub-working module 21 is coupled to one of the plurality of energy interfaces 11.

The main control module 60 has a signal access terminal for accessing the trigger signal, and is configured to control the sub-working module 21 corresponding to the trigger signal to be in a working state to output a driving signal to the connected treatment tip when receiving the trigger signal.

In this embodiment, the energy interface 11 and the sub-working module 21 is implemented using the same devices and modules as in the above embodiment.

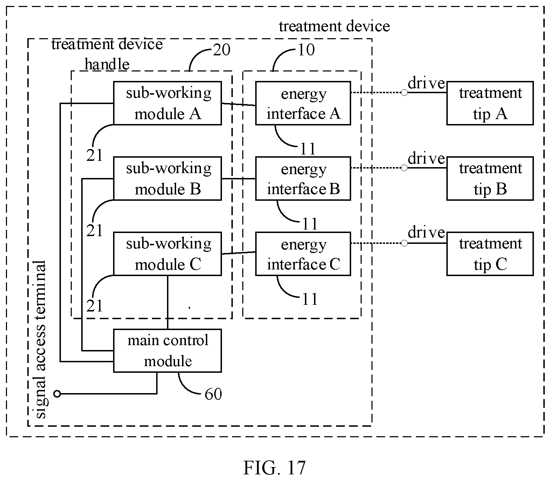

Specifically, as shown in FIG. 17, an embodiment is described as an example, where three energy interfaces 11 in FIG. 14 are provided. The treatment tips are of three kinds ABC, and the sub-working module 21 corresponds to the three kinds ABC.

When the user accesses the treatment tip A, the trigger module 40 connected to the signal access terminal in the above embodiment is operated or the external terminal is operated to send the trigger signal to the communication module 50 connected to the signal access terminal in the above embodiment. When receiving the trigger signal, the main control module 60 controls the sub-working module 21 corresponding to the currently accessed treatment tip to operate, causing the sub-working module 21 to output the driving signal to treatment tip A. For example, the current user has access to an ultrasonic treatment tip. After access, the user triggers button A on the treatment device handle that represents the "ultrasound treatment tip". After receiving the trigger signal from button A, the main control module 60 controls the ultrasonic signal working module 20 corresponding to the ultrasonic treatment tip to start working to output the corresponding ultrasonic signal to the treatment tip A through the energy interface 11A.

It can be understood that the user can also trigger the above-mentioned trigger module 40 or control the external terminal to cause the main control module 60 to stop the current sub-working module 21 from working, or to adjust the intensity of the driving signal, etc.

In another embodiment, the interface module 10 includes at least one energy interface 11, and each energy interface 11 is configured to access at least two kinds of treatment tips.

The working module 20 includes a switch module 22 and a plurality of sub-working modules 21. The switch module 22 is coupled to the output ends of the plurality of sub-working modules 21 and at least one energy interface 11 respectively.

The main control module 60 has a signal access terminal for accessing the trigger signal, and is configured to control the operation of the switch module 22 when receiving the trigger signal to conduct the path between the sub-working module 21 corresponding to the treatment tip and the energy interface 11, and to control the sub-working module 21 corresponding to the trigger signal to be in a working state to output a driving signal to the connected treatment tip.

In this embodiment, the switch module 22 is composed of multiple switch tubes, such as IGBT, MOS, transistor, etc., or is implemented by using a gate chip. The signal terminals of at least two treatment tips of different kinds are completely the same and is connected to the same energy interface 11.

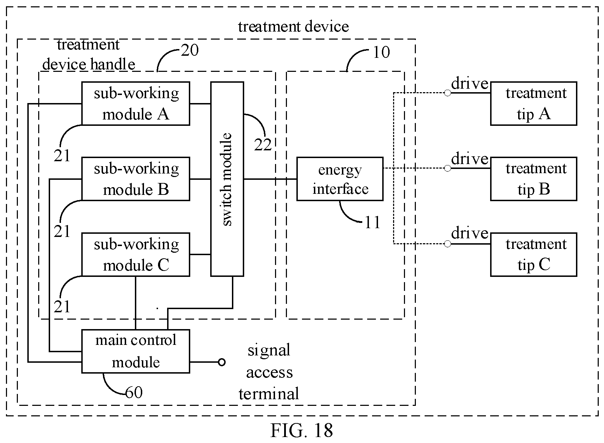

Specifically, as shown in FIG. 18, a case is described as an example, where the treatment tips in FIG. 18 are of three kinds ABC, the sub-working modules 21 correspond to the three kinds ABC, and three kinds of the treatment tips are signal ends corresponding to the same energy interface 11.

When the user connects the treatment tip C to the treatment device handle. Similarly, the user operates the trigger module 40 connected to the signal access terminal in the above embodiment or operate the external terminal to send the trigger signal to the communication module 50 connected to the signal access terminal in the above embodiment. After receiving the trigger signal, the main control module 60 controls the action of the switch module 22 to conduct the path between the sub-working module 21C and the energy interface 11, and control the sub-working module 21C to start working to output the corresponding driving signal to the connected treatment tip C through the switch module 22 and the energy interface 11.

It can be understood that the user can also trigger the above-mentioned trigger module 40 or control the external terminal to cause the main control module 60 to stop the current sub-working module 21 from working, or to adjust the intensity of the driving signal, etc.

In another embodiment, the interface module 10 includes at least one energy interface 11. The working module 20 includes a drive module 23 and a switch module 22, and the switch module 22 is coupled to the output end of the drive module 23 and at least one energy interface 11 respectively.

The main control module 60 has a signal access terminal for accessing a trigger signal, and is configured to control the operation of the switch module 22 to conduct the path between the energy interface 11 corresponding to the treatment tip and the drive module 23, and control the drive module 23 to be in a working state to output the corresponding driving signal to the connected treatment tip.

In this embodiment, the drive module 23 is implemented using the adjustable power module in the above embodiment to provide corresponding operating voltages for different kinds of treatment tips, or is implemented using the integrated chip that can output different driving signals at the same time, such as the signal drive chip that can output ultrasonic signals and radio frequency signals.

Specifically, as shown in FIG. 19, an embodiment is described as an example, where the treatment tips in FIG. 19 are of three kinds ABC and the energy interfaces 11 correspond to three kinds ABC.

When the user connects the treatment tip C to the treatment device handle. Similarly, the user operates the trigger module 40 connected to the signal access terminal in the above embodiment or operate the external terminal to send the trigger signal to the communication module 50 connected to the signal access terminal in the above embodiment. After receiving the trigger signal, the main control module 60 controls the action of the switch module 22 to conduct the path between the drive module 23 and the energy interface 11, and control the drive module 23 to output the drive signal corresponding to the treatment tip C, so that the driving signal is output to the treatment tip C after passing through the energy interface 11C to cause the treatment tip C to start working.

It can be understood that the user can also trigger the above-mentioned trigger module 40 or control the external terminal to cause the main control module 60 to stop the current sub-working module 21 from working, or to adjust the intensity of the driving signal, etc.

The present application proposes a control method for the treatment device handle. The treatment device handle includes an interface module and a working module.

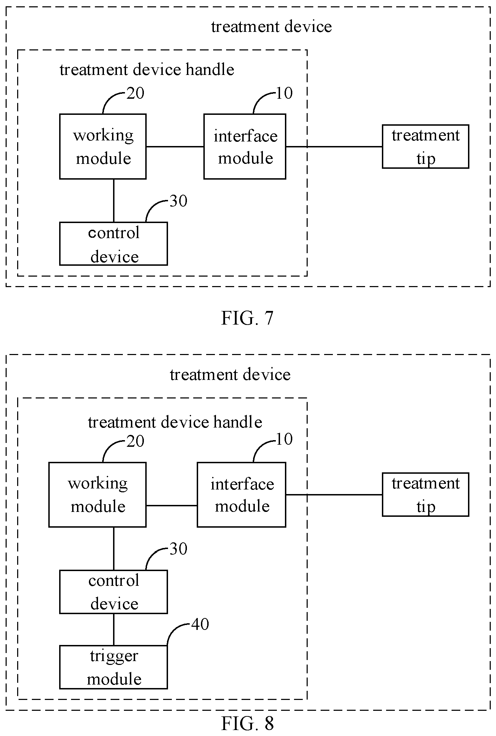

As shown in FIG. 7, it can be understood that a control device for storing and executing the above control method is also provided in the treatment device handle. The control device is implemented by a main controller, such as MCU, DSP, FPGA, PLC, SOC, etc.

In this embodiment, the installation structure is provided on the treatment device handle for installing a fixedly connected treatment tip. The interface module 10 is provided in the mounting structure. When the treatment tip is connected to the installation structure, multiple connection terminals on the treatment tip is connected to the interface module 10 to realize electrical connection with each other.

In an embodiment, the interface module 10 is provided with a plurality of the above-mentioned connectors or conductive parts and other connecting devices for realizing electrical connection functions. Each connecting device is connected to a kind of treatment tip.

In an embodiment, the number of devices such as connectors or conductive parts used to implement electrical connection functions in the interface module 10 may be at least one and no more than the number of kinds of treatment tips. In other words, the structures of the connections for accessing the interface module 10 on some treatment tips of different kinds are exactly the same, that is, the treatment tips of these two kinds share a certain connection device on the interface module 10, thereby effectively simplifying the structure of the treatment device handle, and reducing the cost and process difficulty for manufacturing the treatment device handle.

It is understandable that there are often many kinds of treatment tips in the treatment device, such as ultrasonic treatment tips, radio frequency treatment tips, and light treatment tips.

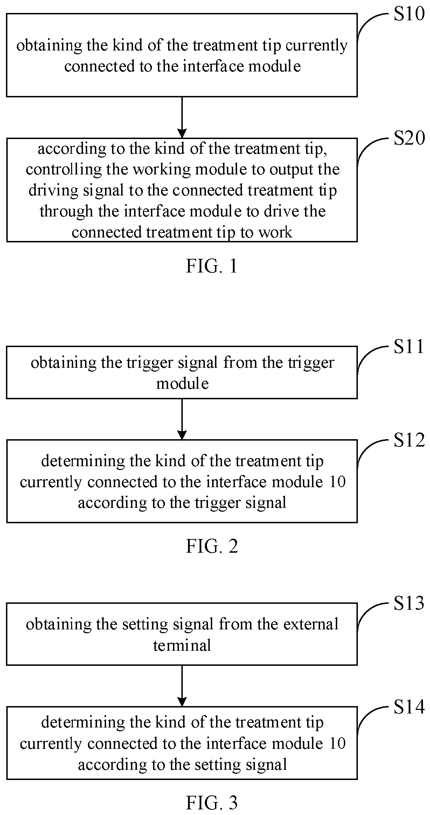

As shown in FIG. 1 and FIG. 7, in an embodiment of the present application, the method includes:
Step S10, obtaining the kind of the treatment tip currently connected to the interface module 10.

In this embodiment, the control device obtains the kind of the treatment tip currently connected to the interface module 10 according to the received external signal.

Specifically, in one embodiment, as shown in FIG. 2, the treatment device handle also includes a trigger module, and obtaining the kind of each treatment tip currently connected to the interface module 10 includes:
Step S11, obtaining the trigger signal from the trigger module; and
Step S12, determining the kind of each treatment tip currently connected to the interface module 10 according to the trigger signal.

In this embodiment, as shown in FIG. 8, the trigger module 40 is coupled to the control device 30 and is implemented using a touch screen, keys, key arrays, etc. After connecting the treatment tip to the interface module 10, the user operates the trigger corresponding to the current treatment tip on the trigger module 40, so that the trigger module 40 outputs the corresponding trigger signal to the control device 30. After receiving the trigger signal, the control device 30 determines the kind of treatment tip currently connected to the interface module 10 according to the trigger signal and the trigger signal-treatment tip kind mapping table preset by the developer.

It can be understood that in addition to manipulating the trigger module 40 on the treatment device handle to achieve settings, the user can also set the kind of connected treatment tip by manipulating the external terminal that is connected to the treatment device handle. Specifically, in another embodiment, as shown in FIG. 3, obtaining the kind of the treatment tip currently accessed to the interface module 10 includes:
Step S13, obtaining the setting signal from the external terminal;
Step S14, determining the kind of the treatment tip currently connected to the interface module 10 according to the setting signal.

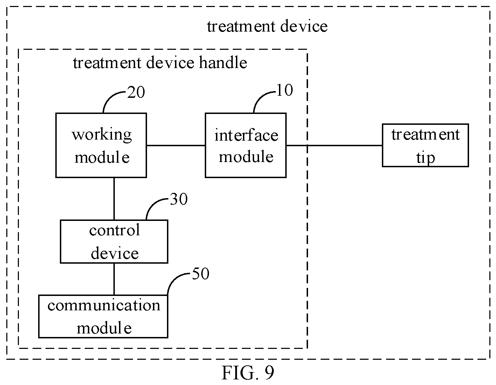

In this embodiment, as shown in FIG. 9, a communication module 50 coupled to the control device 30 can also be provided in the treatment device handle. The communication module 50 is a wireless communication module, such as a WIFI communication module 50, 4G/5G communication module 50, or is a wired communication modules, such as CAN communication module 50, RS485 communication module 50, etc. The control device 30 establishes communication connections with external terminals, such as mobile phones, tablets, terminals, etc., through the communication module 50.

After the user connects the treatment tip to the interface module 10, the user operates the external terminal, for example, touch the corresponding functional area on the APP corresponding to the treatment device handle on the mobile phone, so that the external terminal outputs the corresponding setting signal. After receiving the setting signal through the communication module 50, the control device 30 determines the kind of treatment tip currently connected to the interface module 10 based on the current setting signal and the setting signal-treatment tip kind mapping table preset by the developer.

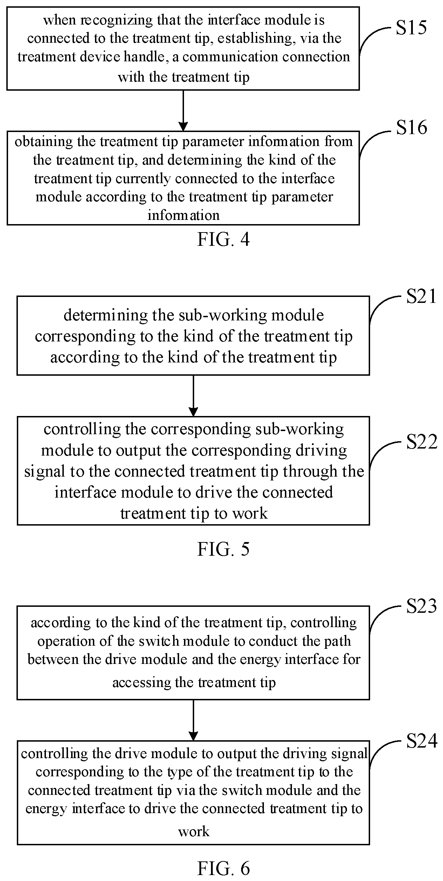

In another embodiment, the control device can also communicate with the connected treatment tip on its own to obtain the kind of the currently connected treatment tip from the connected treatment tip. Specifically, as shown in FIG. 4, obtaining the kind of the treatment tip currently accessed to the interface module 10 includes:
Step S15, when recognizing that the interface module 10 is connected to the treatment tip, establishing, via the treatment device handle, a communication connection with the treatment tip; and
Step S16, obtaining the treatment tip parameter information from the treatment tip, and determining the kind of the treatment tip currently connected to the interface module 10 according to the treatment tip parameter information.

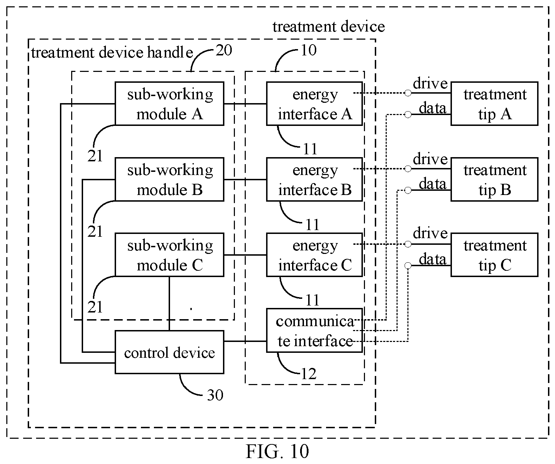

In this embodiment, as shown in FIG. 10 to FIG. 12, the interface module 10 is provided with a communication interface 12. The communication interface 12 is coupled to the control device 30. When the treatment tip is connected, the communication interface 12 establishes an electrical connection with the communication end of the treatment head, so that the control device establishes a communication connection with the treatment head via the communication interface.

It can be understood that multiple communication interfaces is provided and correspond to multiple kinds of treatment tips one-to-one, or one or more communication interfaces is compatible with the access of communication ends of at least two kinds of treatment tips.

Specifically, the control device 30 outputs an inquiry signal through the communication interface at regular intervals. When the treatment tip is connected, the control circuit in the treatment tip receives the inquiry signal through its own communication terminal and send back a feedback signal to allow the control device 30 to determine that the current treatment tip has been accessed. At this time, the control device 30 performs handshake communication with the treatment tip to realize data transmission, and obtain the treatment tip parameter information from the treatment tip to confirm the kind of the currently connected treatment tip based on the treatment tip parameter information. In this way, the user does not need to perform additional operations to make the treatment device handle confirm the kind of treatment tip currently connected, which effectively improves the user's convenience in using the treatment device handle.

Step S20, according to the kind of the treatment tip, controlling the working module to output the driving signal to the connected treatment tip through the interface module 10 to drive the connected treatment tip to work.

In this embodiment, the working module 20 includes a variety of corresponding sub-working modules 21 according to the actual kind of the treatment tip, such as at least two of an ultrasonic energy output module, a radio frequency energy output module, a low frequency energy output module, and a medium frequency energy output module and the light energy output module. As shown in FIG. 5, according to the kind of treatment tip, controlling the working module to output the driving signal to the connected treatment tip through the interface module, so as to drive the connected treatment tip to work specifically includes:
Step S21, determining the sub-working module corresponding to the kind of the treatment tip according to the kind of the treatment tip; and
Step S22, controlling the corresponding sub-working module to output the corresponding driving signal to the connected treatment tip through the interface module to drive the connected treatment tip to work.

When the treatment tip is connected to the interface module 10, the control device 30 determines the sub-working module corresponding to the current treatment tip according to the kind of treatment tip, and control the sub-working module corresponding to the kind of connected treatment tip to work, to output the corresponding driving signal to the treatment tip to make the connected treatment tip work. For example, if an ultrasonic probe is connected, the control device 30 controls the ultrasonic energy output module to output an ultrasonic driving signal to the ultrasonic probe through the interface module 10, so that after receiving the ultrasonic driving signal, the ultrasonic probe emits the corresponding ultrasonic waves to act on the human body.

Specifically, in an embodiment, as shown in FIG. 10, the interface module 10 includes multiple energy interfaces 11. Each of the energy interfaces 11 is configured to access a kind of treatment tip, and the multiple energy interfaces 11 is coupled to multiple sub-working modules 21 respectively. After determining the current kind of the treatment tip, the control device 30 controls the corresponding sub-working module 21 to output the corresponding driving signal to the treatment tip through the energy interface 11 corresponding to its electrical connection, so as to drive the connected treatment tip to work.

In another embodiment, at least one energy interface 11 in the interface module 10 can access at least two kinds of treatment tips. In other words, two kinds of treatment tips is connected to the same energy interface 11, and the plurality of sub-working modules 21 are coupled to the corresponding energy interface 11. When the control device 30 determines the current kind of the treatment tip, it controls the corresponding sub-working module 21 to output the corresponding driving signal to the treatment tip through the energy interface 11 corresponding to its electrical connection to drive the connected treatment tip to work. At the same time, other sub-working modules 21 are controlled to remain in a stopped working state.

In another embodiment, the working module 20 can also be directly implemented using the adjustable power module, such as a programmable power chip, a DC-DC circuit composed of multiple switch tubes, etc. When the treatment tip is connected to the interface module 10, the control device 30 controls the adjustable power module to output the working voltage (i.e., the driving signal) corresponding to the kind of the treatment tip to the treatment tip according to the current kind of the treatment tip and the preset kind of the treatment tip-working voltage mapping table, to enable the treatment tip to be powered on. For example, the working voltage required by the currently connected light treatment tip is 12V. After the light treatment head is connected, the control device 30 controls the adjustable power module to output a working voltage of 12V to the light treatment head through the interface module 10, to power the light treatment head. At this time, the user operates the trigger on the light treatment head to cause the light treatment head to send the corresponding light signal to act on the human body.

In this way, through the above settings, with reference to FIG. 22 to FIG. 24, the treatment device handle 200 of the present application is compatible with different kinds of treatment tips 100, and after the different kinds of treatment tips 100 are connected, the control working module 20 drives different treatment heads 100 to make the treatment tip 100 work, thereby bringing different treatment experiences to the user. In this way, in practical applications, users only need to purchase the treatment device handle 200 and multiple different kinds of treatment tips 100 matched with this handle in the present application to switch between different treatment methods without purchasing a separate set of treatment devices for each treatment method, which greatly improves the user's experience of using the treatment tip 100.

In addition, in an embodiment of the present application, before obtaining the kind of treatment tip currently connected to the interface module, the method further includes:
obtaining access status information of the treatment tip currently connected to the interface module;
according to the access status information, when it is determined that the treatment tip is completely connected to the interface module, obtaining the kind of the treatment tip currently connected to the interface module; or
according to the access status information, when it is determined that the treatment tip is not completely connected to the interface module, prompting or controlling the working module to stop working.

In this embodiment, a trigger module can also be provided on the treatment device handle, and the trigger module 40 is coupled to the control device 30. When the interface module is connected to the treatment tip, the trigger module 40 is triggered, so that the trigger module 40 outputs an access signal to the control device 30. The control device 30 obtains the access status information of the current treatment head according to the currently received access signal.

Specifically, in an embodiment, the trigger module 40 is a contact trigger module, such as a micro switch, etc., and the trigger module 40 is provided in the installation structure of the treatment device handle. When the treatment tip is stably connected to the installation structure, and the signal terminal on the treatment tip is stably coupled to the interface module 10, the treatment tip presses the trigger module 40 provided in the installation structure, so that the trigger module 40 outputs the access signal to the control device 30. For example, the trigger module 40 includes a micro switch. One end of the micro switch is grounded, and the other end is coupled to a pull-up circuit and the control device 30 respectively. When the micro switch is triggered, a low level signal is output to the control device 30, and when the micro switch is not triggered, a high level signal is output to the control device 30.

In an embodiment, the trigger module 40 is also a non-contact trigger module, such as a Hall sensor module. A magnet is provided at the bottom of the treatment tip. When the treatment tip is connected to the installation structure, the Hall sensor module detects the magnetic field formed by the gradually approaching magnet on the treatment head and output an access signal with a corresponding voltage value. The control device 30 confirms that the current treatment tip has been fully inserted into the treatment device handle when the voltage value of the access signal reaches a preset voltage threshold. The preset voltage threshold is obtained by the research and development personnel through multiple tests during the research and development period.

When the control device 30 obtains the access status information of the current treatment tip according to the process of the above embodiment, it performs corresponding actions according to the current access status information. Specifically, if it is determined that the current access status information is that the treatment tip is fully connected, the control device 30 starts to perform the above step 10, that is, start to obtain the kind of the currently connected treatment tip and control the working module 20 to work according to the kind of the treatment tip.

At the same time, a prompt module can also be provided on the treatment device handle. The prompt module is a light signal prompt module, such as an LED light, a display screen, etc., or is a sound prompt module. Specifically, if it is determined that the current access status information indicates that the treatment tip is not fully connected, the control device 30 controls the prompt module to maintain the prompt state. For example, the prompt module is an LED light. When the treatment tip is not connected, the control device 30 controls the LED light to remain on, and when it is confirmed that the treatment tip is connected, the control device 30 controls the LED light to turn off. This serves the purpose of prompting the user whether the treatment tip is currently inserted normally.

In addition, it can be understood that if the current control device 30 confirms that the treatment tip is not fully connected according to the access status information, it also controls the working module to stop working. For example, the current control device 30 outputs a low-level signal to the enable end of the working module, to keep the working module in the stopped working state. Thus, in practical applications, if the user mistakenly triggers the trigger switch on the treatment device handle that is coupled to the control device 30 when the treatment tip is not connected, or mistakenly clicks on the functional area indicating "start working" on the external terminal, so that the external terminal emits a signal indicating "start working" to the treatment device handle, and the working module 20 in the treatment device handle will not start, that is, no electrical signal is output through the energy interface 11, effectively ensuring safety of users when using the treatment device.

The control method for the treatment device handle of the present application includes: obtaining the kind of treatment tip currently connected to the interface module; and controlling the working module to output the driving signal to the connected treatment tip through the interface module to drive the connected treatment tip to work according to the kind of treatment tip. In this way, in practical applications, users can access and drive different kinds of treatment tips through the treatment device handle to experience different kinds of treatment methods without purchasing an additional set of treatment devices. The present application realizes the compatibility of the treatment device handle with different kinds of treatment tips, thereby effectively improving the user's experience and convenience in using the treatment device.

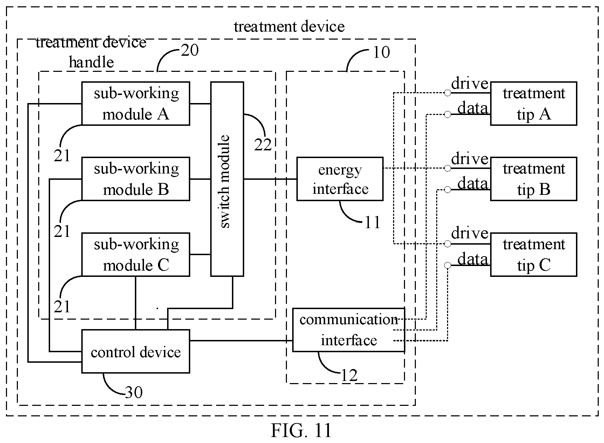

In an embodiment of the present application, as shown in FIG. 11, the interface module 10 includes at least one energy interface 11 for accessing at least two kinds of treatment tips, and the working module 20 also includes a switch module.

It can be understood that in this embodiment, the same energy interface 11 is also used for at least two different kinds of treatment tips. In this way, in the structural design of the interface module 10 of the treatment device handle, the number of energy interfaces 11 is effectively reduced and the integration of the structure is improved.

In this embodiment, the switch module 22 is composed of multiple switch tubes, such as IGBT, MOS, transistor, etc., or is implemented using a gate chip.

Specifically, controlling the corresponding sub-working module to output the corresponding driving signal to the connected treatment tip via the interface module 11 to drive the connected treatment tip to work specifically includes:
according to the kind of treatment tip, controlling the operation of the switch module to conduct the path between the sub-working module corresponding to the kind of treatment tip and the energy interface for accessing the treatment tip; and
controlling the corresponding sub-working module to output the corresponding driving signal to the connected treatment tip through the switch module and the energy interface to drive the connected treatment tip to work.

As shown in FIG. 11, an embodiment is described as an example, where the treatment tip in FIG. 11 is of three kinds ABC, the sub-working module 21 corresponds to the three kinds ABC, and the energy interface 11 is compatible with the three kinds of the treatment tips.

If the current user wants to use treatment tip B for treatment, treatment tip B is connected to the installation structure of the treatment device handle. At this time, the control device 30 confirms that the kind of the currently connected treatment tip is treatment tip B, and then determine that the currently required sub-working module is sub-working module B according to the preset kind of the treatment tip-sub-working module mapping table. Subsequently, the control device 30 controls the action of the switch module 22 to conduct the path between the sub-working module 21B and the energy interface 11, and control the sub-working module 21B to output the corresponding driving signal to the connected treatment tip B via the switch module and the energy interface, to drive the connected treatment tip B to work.

In this way, through the above arrangement, it is possible to save space for providing multiple energy interfaces on the treatment device handle, and effectively improve the integration of the treatment device handle.

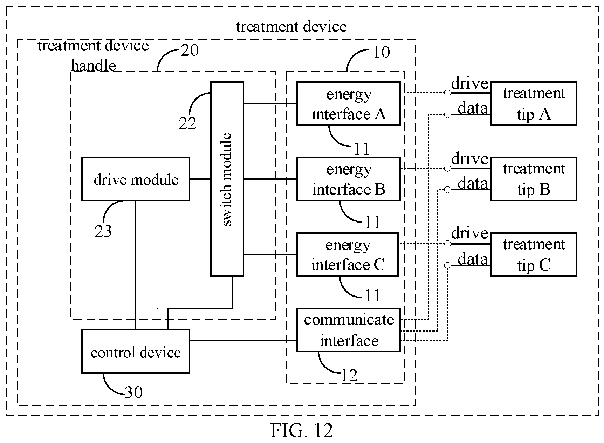

In an embodiment of the present application, with reference to FIG. 6 and FIG. 12, the interface module includes at least one energy interface for accessing the treatment tip. The working module includes a switch module and a drive module. According to the kind of treatment tip, controlling the working module to output the driving signal to the connected treatment tip via the interface module to drive the connected treatment tip to work specifically includes:
Step S23, according to the kind of the treatment tip, controlling operation of the switch module to conduct the path between the drive module and the energy interface for accessing the treatment tip; and
Step S24, controlling the drive module to output the driving signal corresponding to the kind of the treatment tip to the connected treatment tip via the switch module and the energy interface to drive the connected treatment tip to work.

In this embodiment, the drive module 23 is implemented using the adjustable power module in the above embodiment to provide corresponding operating voltages for different kinds of treatment tips, or is implemented using the integrated chip that can output different driving signals at the same time, such as signal driver chips that can output ultrasonic signals and radio frequency signals.

It can be understood that in this embodiment, the number of energy interfaces is consistent with the number of treatment tips that is connected, that is, each energy interface is configured to access one kind of treatment tip. Alternatively, the number of energy interfaces may be less than the number of treatment tips, that is, at least one energy interface can access at least two kinds of treatment tips.

In this embodiment, after obtaining the kind of the current treatment tip, the control device 30 determines the energy interface 11 currently connected to the treatment tip, and control the current switch module to conduct the path between the energy interface 11 and the output end of the drive module 23. Subsequently, the drive module 23 is controlled to output the driving signal corresponding to the kind of the treatment tip to the connected treatment tip via the switch module and the energy interface to drive the connected treatment tip to work.

Specifically, as shown in FIG. 12, an embodiment is described as an example, where the treatment tip in FIG. 12 are of three kinds ABC and the energy interfaces 11 correspond to three kinds ABC.

When the user wants to use the treatment tip C, he connects the treatment tip C to the installation structure of the treatment device handle. At this time, communication is established between the treatment tip C and the control device 30 via the communication interface 12. The control device 30 determines that the connected treatment tip is the treatment tip C, control the action of the switch module 22 to conduct the path between the drive module 23 and the energy interface C11, and control the drive module 23 to output the driving signal corresponding to the treatment tip C through the switch module 22 and the energy interface C11 to the treatment tip to drive the treatment tip to work.

In this way, through the above arrangement, the wiring area of the relevant circuits of the internal working module of the treatment device handle is reduced, the space within the treatment device handle is saved, and the structural integration is improved.

The present application also proposes a control device, including a memory, a processor, and a control program for the treatment device handle stored in the memory and executed by the processor. When the control program for the treatment device handle is executed by the processor, the control method for the treatment device handle as described above is implemented.

It is worth noting that since the control device of the present application includes all the technical solutions of the embodiments of the above-mentioned control method for the treatment device handle, it at least has all the beneficial effects brought by the technical solutions of the above-mentioned control method for the treatment device handle, which is not repeated here.

The present application also proposes a treatment device handle, including the control device as described above.

It is worth noting that since the treatment device handle of the present application includes all the technical solutions of the embodiments of the above control device, it has at least all the beneficial effects brought by the technical solutions of the above control device, which will not be repeated here.

As shown in FIG. 22 to FIG. 24, the present application also proposes a treatment device, including the treatment device handle 200 as described above and at least two different treatment tips 100.

In this embodiment, the treatment tip 100 is at least two of an ultrasonic treatment tip, a radio frequency treatment tip, a low frequency treatment tip, a medium frequency treatment tip and a light treatment tip.

It is worth noting that since the treatment device of the present application includes all the technical solutions of the embodiments of the above-mentioned treatment device handle, it has at least all the beneficial effects brought by the above-mentioned technical solution of the treatment device handle, which will not be repeated here.

The above are only some embodiments of the present application, and are not intended to limit the scope of the present application. Under the inventive concept of the present application, equivalent structural transformations made by using the contents of the description and drawings of the present application, or direct/indirect application in other related technical fields, are included in the scope of the present application.

## Claims

1. A treatment device handle, **characterized by** comprising:
an interface module, configured to access at least one kind of treatment tip;
a working module coupled to the interface module; and
a main control module coupled to the working module, wherein the main control module is configured to control the working module to drive the at least one kind of treatment tip through the interface module.

2. The treatment device handle according to claim 1, wherein the interface module comprises at least one communication interface and at least one energy interface;
the energy interface is coupled to the working module and is configured to access a signal terminal of each treatment tip; and
the communication interface is coupled to the main control module and is configured to access a communication terminal of the treatment tip; and in response to that the interface module is connected with each treatment tip, the communication terminal of each treatment tip is configured to establish an electrical connection path with the main control module through the communication interface.

3. The treatment device handle according to claim 2, wherein a plurality of the energy interfaces are provided, and each energy interface is configured to access one kind of treatment tip; and
the working module comprises a plurality of sub-working modules, and an output end of each sub-working module is coupled to one of the plurality of energy interfaces.

4. The treatment device handle according to claim 2, wherein at least one energy interface is provided, and each energy interface is configured to access at least two kinds of treatment tips;
the working module comprises a switch module and a plurality of sub-working modules; the switch module is coupled to output ends of the plurality of sub-working modules and at least one energy interface, and a controlled terminal of the switch module is coupled to the main control module; or
the working module comprises a plurality of sub-working modules, and the plurality of sub-working modules are coupled to the main control module.

5. The treatment device handle according to claim 2, wherein the working module comprises a drive module and a switch module; the switch module is coupled to an output end of the drive module and at least one energy interface respectively, and a controlled end of the switch module is coupled to the main control module.

6. The treatment device handle according to claim 5, wherein the main control module is provided with a signal access terminal for accessing a trigger signal; and
the main control module is configured to control a working state of the drive module in response to receiving the trigger signal.

7. The treatment device handle according to claim 3 or 4, wherein the main control module is provided with a signal access terminal for accessing a trigger signal; and
the main control module is configured to control a working state of a sub-working module corresponding to the treatment tip in response to receiving the trigger signal.

8. The treatment device handle according to claim 1, wherein the interface module comprises a plurality of energy interfaces, and each of the energy interfaces is configured to access one kind of treatment tip;
the working module comprises a plurality of sub-working modules, and an output end of each sub-working module is coupled to one of the plurality of energy interfaces; and
the main control module is provided with a signal access terminal for accessing a trigger signal, and is configured to control a working state of a sub-working module according to the trigger signal in response to receiving the trigger signal.

9. The treatment device handle according to claim 1, wherein the interface module comprises at least one energy interface and the at least one energy interface is configured to access at least two kinds of treatment tips;
the working module comprises a switch module and a plurality of sub-working modules; the switch module is coupled to output ends of the plurality of sub-working modules and the at least one energy interface, and a controlled end of the switch module is coupled to the main control module; and
the main control module is provided with a signal access terminal for accessing a trigger signal, and is configured to control operation of the switch module and control a working state of a sub-working module according to the trigger signal in response to receiving the trigger signal.

10. The treatment device handle according to claim 1, wherein the interface module comprises at least one energy interface; the working module comprises a drive module and a switch module; the switch module is coupled to an output end of the drive module and at least one energy interface respectively, and a controlled end of the switch module is coupled to the main control module; and
the main control module is provided with a signal access terminal for accessing a trigger signal, and is configured to control operation of the switch module and a working state of the drive module in response to receiving the trigger signal.

11. The treatment device handle according to claim 3, wherein the plurality of sub-working modules comprises at least two of an ultrasonic energy output module, a radio frequency energy output module, a low frequency energy output module, a medium frequency energy output module and a light energy output module.

12. The treatment device handle according to claim 1, further comprising:
a trigger module coupled to a signal access terminal of the main control module, wherein in response to that the interface module is connected to the treatment tip, the trigger module is triggered to output a access signal to the main control module; or
the trigger module is configured to output a trigger signal to the main control module in response to that the trigger module is triggered by a user.

13. A control method for a treatment device handle, the treatment device handle comprising an interface module and a working module, and the method comprising:
obtaining kind of each of a plurality of treatment tips currently connected to the interface module; and
according to the kind of each treatment tip, controlling the working module to output a driving signal to the treatment tip through the interface module to drive the treatment tip to work.

14. The control method for the treatment device handle according to claim 13, wherein the treatment device handle further comprises a trigger module, and obtaining the kind of each treatment tip currently connected to the interface module comprises:
obtaining a trigger signal sent from the trigger module;
determining the kind of each treatment tip currently connected to the interface module according to the trigger signal; or
obtaining the kind of each treatment tip currently connected to the interface module comprises:
obtaining a setting signal sent from an external terminal; and
according to the setting signal, determining the kind of each treatment tip currently connected to the interface module.

15. The control method for the treatment device handle according to claim 13, wherein obtaining the kind of the treatment tip currently connected to the interface module comprises:
in response to that the treatment device handle recognizes that the interface module is connected with each treatment tip, establishing a communication connection between the treatment device handle and the treatment tip; and
obtaining treatment tip parameter information from the treatment tip, and determining the kind of each treatment tip currently connected to the interface module according to the treatment tip parameter information.

16. The control method for the treatment device handle according to claim 13, wherein the working module comprises a plurality of sub-working modules; and the according to the kind of each treatment tip, controlling the working module to output the driving signal to the treatment tip through the interface module to drive the treatment tip to work comprises:
according to the kind of each treatment tip, determining the sub-working module corresponding to the kind of each treatment tip; and
controlling the corresponding sub-working module to output a corresponding driving signal to the treatment tip through the interface module to drive the treatment tip to work.

17. The control method for the treatment device handle according to claim 16, wherein the interface module comprises at least one energy interface for accessing at least two kinds of treatment tips, and the working module also comprises a switch module; the controlling the corresponding sub-working module to output the corresponding driving signal to the treatment tip through the interface module to drive the treatment tip to work comprises:
according to the kind of each treatment tip, controlling operation of the switch module to conduct a path between the sub-working module corresponding to the kind of the treatment tip and the energy interface for accessing the treatment tip; and
controlling the corresponding sub-working module to output the corresponding driving signal to the treatment tip through the switch module and the energy interface to drive the treatment tip to work.

18. The control method for the treatment device handle according to claim 13, wherein the interface module comprises at least one energy interface for accessing the treatment tip, and the working module comprises a switch module and a drive module; the according to the kind of each treatment tip, controlling the working module to output the driving signal to the treatment tip through the interface module to drive the treatment tip to work comprises:
according to the kind of each treatment tip, controlling operation of the switch module to conduct a path between the drive module and the energy interface for accessing the treatment tip; and
controlling the drive module to output the driving signal corresponding to the kind of each treatment tip to the treatment tip through the switch module and the energy interface to drive the treatment tip to work.

19. The control method for the treatment device handle according to claim 13, wherein before the obtaining the kind of each treatment tip currently connected to the interface module, the method further comprises:
obtaining access status information of the treatment tip currently connected to the interface module;
according to the access status information, in response to determining that the treatment tip is completely connected to the interface module, obtaining the kind of each treatment tip currently connected to the interface module; or
according to the access status information, in response to determining that the treatment tip is not completely connected to the interface module, prompting or controlling the working module to stop working.

20. A treatment device, **characterized by** comprising a plurality of treatment tips and a treatment device handle according to any one of claims 1 to 12, wherein the plurality of treatment tips are connected to the treatment device handle according to the control method for the treatment device handle according to any one of claims 13 to 19, and an output driving signal is obtained and controlled; the plurality of treatment tips comprises at least two of an ultrasonic treatment tip, a low-frequency treatment tip, a medium-frequency treatment tip and a light treatment tip.
